Europäisches Patentamt

European Patent Office

Office européen des brevets

(19)

(11) Numéro de publication : **0 052 571**
**B1**

(12)                    FASCICULE DE BREVET EUROPÉEN

(45) Date de publication du fascicule du brevet :
**11.11.87**

(21) Numéro de dépôt : **81420160.4**

(22) Date de dépôt : **30.10.81**

(51) Int. Cl.⁴ : **A 61 M   1/14**

(54) **Rein artificiel avec circuit de liquide de dialyse intégré.**

(30) Priorité : **13.11.80 FR 8024476**

(43) Date de publication de la demande :
**26.05.82 Bulletin 82/21**

(45) Mention de la délivrance du brevet :
**11.11.87 Bulletin 87/46**

(84) Etats contractants désignés :
**BE CH DE GB IT LI NL SE**

(56) Documents cités :
**FR-A- 2 082 978**
**US-A- 3 774 762**

(73) Titulaire : **HOSPAL INDUSTRIE**
**7, Avenue Lionel Terray**
**F-69330 Meyzieu (FR)**

(72) Inventeur : **Barthe, Bernard**
**62, rue Pasteur Bondoufle**
**F-91220-Bretigny sur Orge (FR)**
Inventeur : **Vantard, Georges**
**20, rue des Prés de Noisy**
**F-93460-Gournay sur Marne (FR)**
Inventeur : **Vasseur, Jean-Pierre**
**19, Square Maurice Ravel**
**F-91160-Longjumeau (FR)**

(74) Mandataire : **Gauckler, Jacques et al**
**Service Brevets HOSPAL HOSPAL C.O.T. B.P. 21**
**F-69881 Meyzieu Cedex (FR)**

## Description

La présente invention concerne un rein artificiel. Elle concerne plus particulièrement un rein artificiel comportant divers organes incorporés ensemble, de préférence en vue d'un usage unique.

Les reins artificiels actuellement utilisés comportent notamment un hémodialyseur à usage unique, ainsi que des éléments constituant un circuit parcouru par le sang, également à usage unique. Dans le but de simplifier la fabrication de ces dispositifs, de réduire leur coût et de faciliter leur utilisation, on a proposé d'incorporer à l'hémodialyseur certains éléments du circuit parcouru par le sang, formant ainsi un seul ensemble à usage unique autour de l'hémodialyseur, auquel on peut raccorder lors de l'utilisation, outre quelques accessoires, les éléments constituant le circuit parcouru par le liquide de dialyse, qui eux sont généralement réutilisables après stérilisation.

Toujours dans le même but de simplification, de réduction des coûts et d'accroissement de la sécurité, on a proposé dans le US-A-3 774 762 d'intégrer les circuits parcourus par le sang et par le liquide de dialyse en un système à usage unique de deux feuilles en matière plastique, préformées et soudées entre elles, délimitant ainsi lesdits circuits. Ces feuilles sont supportées par deux plaques rigides, d'usage permanent, portant des dispositifs agissant comme pompe et comme vanne. Une armoire regroupe les organes usuels de commande et de contrôle.

Cependant l'ensemble des circuits et des accessoires du rein artificiel étant étalé sur de larges panneaux, un tel dispositif ne présente ni la compacité, ni la maniabilité nécessaires. De plus l'intégration des divers organes reste partielle et ne s'étend pas à l'hémodialyseur lui-même. Enfin il ne comporte pas de moyen pour régler et mesurer directement le volume d'ultrafiltrat retiré au patient.

Aussi est-ce un objet de la présente invention de proposer un nouveau type de construction intégrée pour rein artificiel, plus compacte, permettant d'une part l'exécution de nouvelles fonctions telles que le prélèvement et la mesure de volumes de liquide égaux aux quantités d'ultrafiltrat retiré au patient, permettant d'autre part une intégration plus poussée pouvant s'étendre à l'hémodialyseur lui-même et permettant également un gain sensible de poids, d'encombrement et facilitant le cas échéant son transport, son stockage et son utilisation.

Un autre objet de la présente invention est un rein artificiel dont la mise en œuvre soit rapide, sûre, simple et économique.

D'autres objets de la présente invention apparaîtront au cours de la description qui va suivre.

Il a maintenant été trouvé un rein artificiel comprenant :

a) un hémodialyseur comportant un boîtier séparé en deux compartiments, le premier muni de moyens pour la circulation du sang et le second pour la circulation du liquide de dialyse, par une membrane permettant le traitement du sang par dialyse et par ultrafiltration,

b) des moyens extérieurs audit hémodialyseur pour faire circuler le sang dans ledit premier compartiment,

c) des moyens extérieurs audit hémodialyseur, pour préparer le liquide de dialyse et le faire circuler,

d) des moyens pour stocker ledit liquide de dialyse, frais et/ou usagé,

e) des moyens pour prélever et mesurer des quantités de liquide égales aux quantités d'ultrafiltrat désirées et,

f) des organes de commande et de contrôle desdits moyens selon b) et c),

lesdits moyens selon b, c, d et/ou e étant essentiellement constitués de trois éléments non permanents, à savoir un élément flexible ayant la forme générale d'une feuille plane, localement ajourée, comprise à l'intérieur et maintenue serrée entre deux éléments rigides ou semi-rigides, ces trois éléments coopérant ensemble pour, d'une part délimiter les circuits de liquide entre l'élément flexible et les éléments rigides, à l'intérieur et au contact desdits éléments rigides ou semi-rigides et à l'extérieur dudit élément flexible, lesdits circuits étant susceptibles de communiquer à travers ledit élément flexible, et pour, d'autre part, assurer les étanchéités.

La présente invention n'est pas fondée sur une recherche de la meilleure façon possible d'assembler toutes les pièces nécessaires à la réalisation des fonctions souhaitées, mais au contraire sur une recherche de la réduction du nombre d'organes autonomes par incorporation dans un ensemble commun d'organes différents ayant chacun au moins une fonction particulière.

Selon l'invention on résout ce problème par une intégration au moins partielle des moyens de préparation du liquide de dialyse et de circulation dans et au-delà de l'hémodialyseur. Par intégration on entend ici l'emploi d'éléments multifonctionnels, communs à au moins deux organes différents et ayant dans chacun de ces organes une fonction particulière. Ces fonctions peuvent être soit de même nature, soit de nature différente.

Par intégration totale, on entend un ensemble regroupant divers organes constitués exclusivement d'éléments multifonctionnels.

Par intégration partielle, on entend soit un ensemble constitué d'organes dont une partie seulement a en commun un ou plusieurs éléments multifonctionnels, soit un ensemble constitué d'organes qui ne sont que partiellement constitués d'éléments multifonctionnels, soit encore un ensemble constitué par une combinaison des deux cas précédents.

Par ailleurs, comme les hémodialyseurs ont jusqu'à présent bénéficié de nombreux et importants perfectionnements justifiés par des fabrications en grande série et comme l'appareillage ayant notamment pour fonction de préparer, de stocker et de faire circuler le liquide de dialyse, ainsi que de contrôler l'ultrafiltrat n'a, par contraste, que relativement peu évolué, il en résulte un sensible déséquilibre technologique. La présente invention propose donc de transférer le plus possible d'éléments de cet appareillage vers une unité à usage unique, le plus souvent construite autour de l'hémodialyseur, pour leur faire bénéficier des technologies de grande série.

Il apparaîtra clairement dans la description qui va suivre que le circuit de dialyse d'un rein artificiel étant constitué d'une pluralité d'organes assurant chacun des fonctions très particulières, l'intégration dans un ensemble commun de deux ou de plusieurs de ces organes peut se prêter à de multiples solutions, d'abord par le fait de la nature et du nombre des organes intégrés, ensuite par le choix de la solution technologique retenue pour réaliser cette intégration.

On ne décrira donc ici qu'à titre d'exemple illustratif un mode de réalisation de la présente invention et l'on citera diverses variantes possibles, la portée de la présente invention n'étant en rien limitée ni à cet exemple, ni à ces variantes.

La compréhension de l'invention sera facilitée par les figures ci-jointes qui représentent schématiquement et sans échelle déterminée divers aspects d'un exemple de réalisation. Les mêmes numéros désigneront des organes identiques ou équivalents dans les différentes figures.

La figure 1 est la vue extérieure de l'ensemble du rein artificiel selon un mode de réalisation préféré de la présente invention.

La figure 2 est une vue latérale en coupe partielle du rein artificiel selon la figure 1.

La figure 3 est un schéma des moyens pour préparer, stocker et faire circuler le liquide de dialyse selon l'invention.

La figure 4 est la vue en perspective de la pièce inférieure (61) de l'ensemble commun qui regroupe autour de l'hémodialyseur les moyens pour préparer et faire circuler le liquide de dialyse, ainsi que pour prélever des quantités de liquide égales aux quantités d'ultrafiltrat désirées.

La figure 5 est une vue en perspective de la pièce supérieure (62) dudit ensemble commun selon la figure 4, vue partiellement éclatée.

La figure 6 est la vue en perspective de la pièce intermédiaire (60) dudit ensemble commun, selon les figures 4 et 5, ainsi que d'une pièce annexe (80).

La figure 7 est la vue en coupe par des plans verticaux axiaux de la pompe à membrane (38).

La figure 8 est une vue en coupe par des plans verticaux axiaux du dispositif à flotteur pour la régulation de la pression du liquide de dialyse et du conduit de mise à l'atmosphère de ce dispositif.

La figure 9 est une vue en coupe par des plans verticaux axiaux d'une partie du dispositif à flotteur et des moyens pour prélever et mesurer des quantités de liquide égales aux quantités d'ultrafiltrat désirées.

La figure 10 est une vue en coupe par un plan vertical d'une variante de réalisation du dispositif à flotteur selon la figure 8.

La figure 11 est la vue éclatée en perspective d'un ensemble commun intégré constitué par des éléments multifonctionnels semblables aux éléments (60) et (80), puis (61) et (62) représentés séparément, respectivement figures 6, 4 et 5.

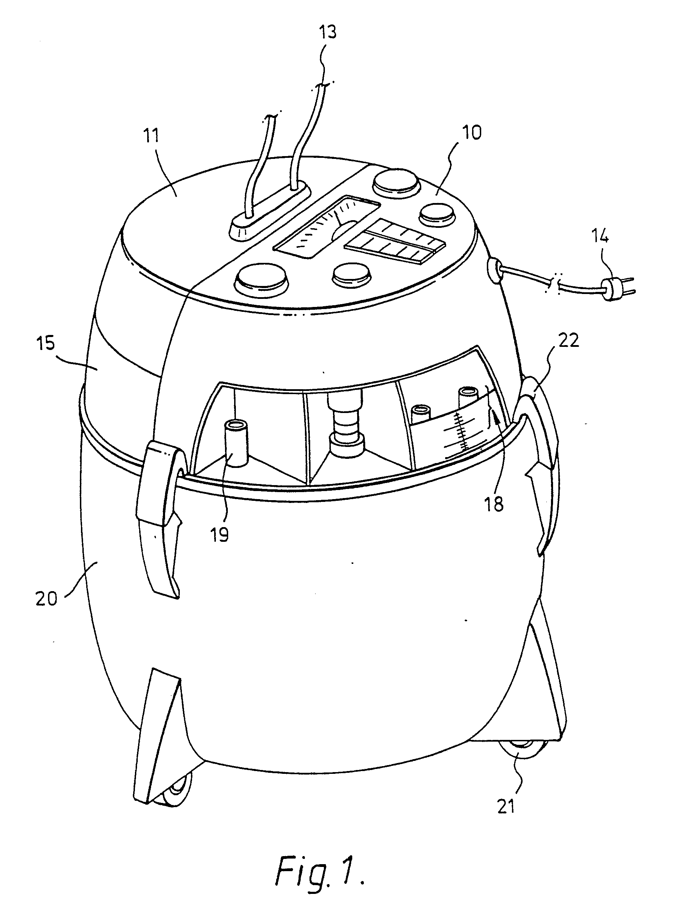

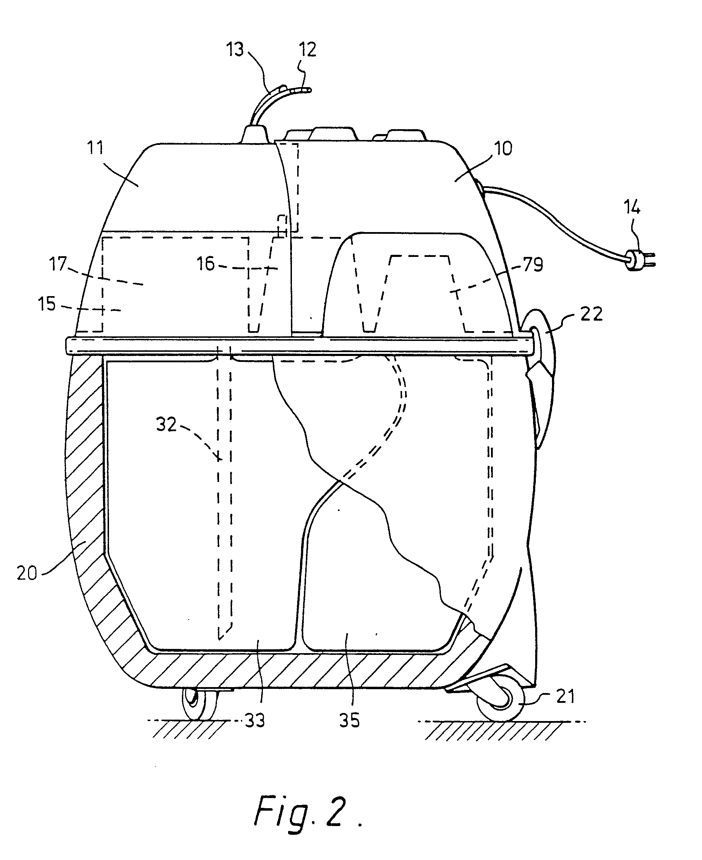

Le rein artificiel représenté figures 1 et 2 peut se subdiviser en trois zones de niveaux différents. La zone supérieure comprend une console (10) groupant les organes de commande et de contrôle du rein artificiel, ainsi que, sous un carénage approprié (11), les moyens extérieurs à l'hémodialyseur pour faire circuler le sang. Les conduits de circulation du sang (12) et (13) relient le rein artificiel au patient ; la console peut être reliée à une source de courant électrique grâce à la prise (14).

Dans la zone intermédiaire (15) sont groupés autour de l'hémodialyseur (16) des moyens (17) pour préparer le liquide de dialyse, des moyens pour le faire circuler, ainsi que des moyens (18) pour prélever et mesurer des quantités de liquide égales aux quantités d'ultrafiltrat désirées. Les moyens (17) peuvent être reliés par l'embout (19) à une source d'alimentation en eau courante, préalablement chauffée et adoucie.

La zone inférieure est constituée par un container (20) destiné à contenir et à délimiter le volume de liquide de dialyse nécessaire à une séance. Il est indéformable sous les contraintes habituelles et ses parois sont de préférence en matériau thermiquement isolant pour conserver le liquide de dialyse à une température voisine de celle du sang pendant la durée de la séance. Avantageusement il peut être monté sur roulettes (21) et il peut être muni de dispositifs de fermeture rapide à levier (22) pour maintenir les principaux éléments du rein artificiel pendant la séance d'hémodialyse.

En procédant à des traitements selon des taux d'épuration relativement modérés, définis par exemple par une clairance hebdomadaire en urée de 70 à 90 litres et en vitamine $B_{12}$ de 20 à 30 litres, on peut réduire le volume total de liquide de dialyse nécessaire à une séance, de 200 à 300 litres environ à moins de 50 litres ; 30 litres par exemple.

Selon l'invention, il est en outre possible, grâce à l'intégration de différents organes du circuit du liquide de dialyse dans un ensemble commun, de réduire le volume de l'appareillage à moins d'une dizaine de litres, de sorte que, au total, le volume de l'ensemble du rein artificiel avec en outre le liquide de dialyse nécessaire à une séance, ne dépasse pas 40 litres environ, contre 400 litres environ pour des reins artificiels conventionnels. Ceci est un avantage considérable pour le transport, le stockage et l'utilisation.

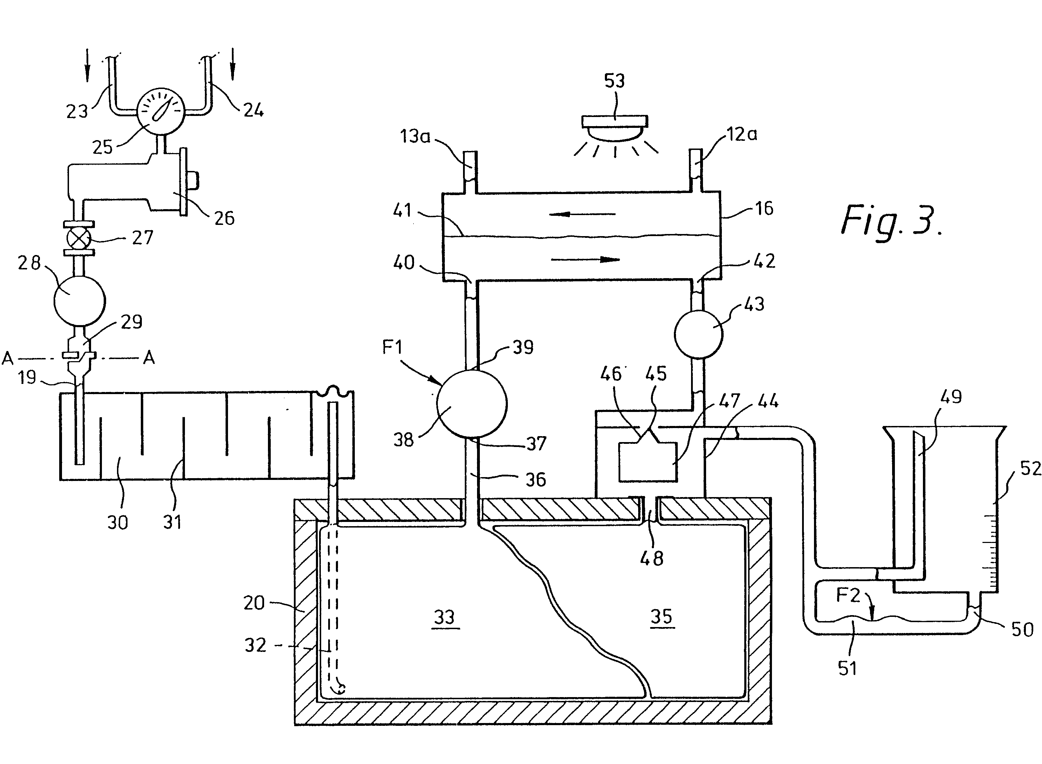

La figure 3 montre des organes nécessaires à la préparation, au stockage et à la circulation du liquide de dialyse dans le rein artificiel selon l'invention, ainsi que leurs principales liaisons. Une partie de ces organes, situés par exemple en amont du trait mixte AA indiquant le milieu du raccord (29) peut être installé à demeure et être réutilisé à chaque séance d'hémodialyse. Les organes disposés à l'aval de ce raccord sont, selon l'invention, au moins partiellement intégrés pour former un ensemble commun. Avantageusement, les organes disposés à l'aval de ce raccord peuvent être, de préférence en presque totalité, à usage unique.

Le circuit de distribution d'eau courante comprend une arrivée d'eau froide (23) et une arrivée d'eau chaude (24). Un dispositif mélangeur (25) muni d'un thermostat d'un type disponible dans le commerce, permet de fournir une eau à des températures comprises entre 37 °C et 40 °C.

Le cas échéant, cette eau traverse un dispositif adoucisseur (26) muni par exemple de résines échangeuses d'ions de types connus en soi, régénérables périodiquement. L'eau adoucie traverse ensuite un robinet à commande manuelle (27) et une vanne automatique (28) dont la fermeture est commandée par un pressostat limitant la pression de l'eau à l'aval à une valeur réglée à l'avance, de l'ordre de 100 mm de mercure (0,13 bar).

Un raccord rapide (29) de type connu en soi permet de relier les organes amont réutilisables, aux organes aval, le plus souvent à usage unique.

Ce raccord rapide (29) est relié, par l'intermédiaire d'un embout (19), à un réservoir (30) contenant initialement de préférence une solution concentrée de liquide de dialyse. Le volume de ce réservoir est déterminé pour contenir à l'avance la quantité de concentré nécessaire correspondant exactement à la capacité en liquide de dialyse du circuit. A une capacité en liquide de dialyse de 30 litres correspond un volume de solution concentrée d'environ 1 litre qui doit être défini avec une précision de + 25 ml. Le réservoir (30) est avantageusement muni de chicanes (31) qui facilitent le déplacement par l'eau de la solution concentrée.

Un tube plongeur (32) relie la partie supérieure du réservoir (30) au fond du container (20). Avantageusement l'eau et le concentré de dialyse sont recueillis à l'intérieur du premier des deux compartiments (33), (35) d'une poche plastique souple, déformable, étanche, initialement vide d'air et susceptible d'occuper un volume égal au volume interne du container.

Le container (20) est fermé par un couvercle (34) qui peut être traversé par des canalisations telles que le tube plongeur (32). Le compartiment (33) peut être refermé autour du tube plongeur (32), par exemple thermoscellé sous le couvercle. Il se raccorde aussi de manière étanche au tube (36) relié à l'orifice d'aspiration (37) de la pompe (38).

Celle-ci peut être du type pompe à membrane et être actionnée selon la flèche $F_1$ par tout dispositif de type connu en soi (non représenté) tel qu'une came commandée par un moteur électrique.

L'orifice de refoulement (39) est relié à l'orifice (40) de l'hémodialyseur (16). Celui-ci est séparé par une membrane (41) en deux compartiments : le premier compartiment parcouru par le sang entre les orifices (12a) et (13a) sur lesquels se raccordent les conduits (12) et (13), le second compartiment parcouru par le liquide de dialyse, généralement à contre courant du sang, entre les orifices (40) et (42).

Le cas échéant, un dispositif de chauffage (53), par exemple à rayonnement infra-rouge, solidaire de la console (10), maintient la température du sang à la sortie de l'hémodialyseur dans l'intervalle désiré.

L'orifice (42) de l'hémodialyseur est relié à un élément de conduit transparent (43) coopérant avec un colorimètre d'un type connu en soi (non représenté), logé dans la console (10). Cet élément (43) est relié à un bac (44) muni à la partie supérieure d'un orifice (45), siège du pointeau (46) d'un flotteur (47), mobile à l'intérieur du bac (44). Le fond du bac (44) s'ouvre par un orifice (48) directement sur l'intérieur du container (20). Le bac (44) respire à l'atmosphère par un conduit (49).

Le liquide de dialyse usagé peut s'accumuler dans le container (20), à l'intérieur du compartiment (35) de la poche souple, semblable au compartiment (33) et complémentaire de celui-ci.

Le dispositif obturateur constitué par le pointeau solidaire du flotteur se déplaçant face à son siège (45) régule automatiquement à tout instant la pression du liquide de dialyse dans l'hémodialyseur à des valeurs comprises entre la pression atmosphérique et la pression du sang.

Les moyens pour prélever et mesurer des quantités (en volume, en poids et/ou en débit) de liquide égales aux quantités d'ultrafiltrat désirées sont constitués par un conduit (50) muni d'une section souple (51) susceptible d'être fermée par un dispositif extérieur de tout type connu tel qu'une vis réglable qui agit selon la flèche $F_2$ pour obturer plus ou moins la section du conduit (50). Ce conduit (50) s'ouvre dans un récipient gradué (52). Avantageusement le conduit (49) débouche également dans le récipient (52). Le volume de liquide soutiré est aussitôt remplacé par un égal volume d'ultrafiltrat traversant la membrane jusqu'à ce que la pression du liquide de dialyse dans l'hémodialyseur soit rétablie à sa valeur initiale. Le rein artificiel selon l'invention contrôle donc l'ultrafiltrat préférentiellement par une méthode volumétrique.

Selon un mode de réalisation particulier de l'invention, on a trouvé que l'on peut intégrer dans un ensemble commun les organes suivants :

le bac (30) contenant initialement une quantité calibrée de solution concentrée de liquide de dialyse,

la pompe (38),

l'hémodialyseur (16),

le conduit transparent (43),

le bac (44) et son flotteur (47),

le récipient gradué (52), les conduits (49) et (50) et le dispositif de fermeture (51).

Cet ensemble commun peut être relié d'une part par le raccord (29) à une source d'eau préalablement chauffée et adoucie et d'autre part à un container de stockage (20) du volume de liquide de dialyse exactement nécessaire à une séance d'hémodialyse. De préférence, le container (20) est lui-même intégré à l'ensemble commun défini ci-avant.

On a trouvé que l'on pouvait avantageusement rendre multifonctionnelles différentes pièces de l'hémodialyseur, telles qu'un joint d'étanchéité et des pièces de contention.

Cette intégration n'est pas une simple juxtaposition d'organes connus dont on aurait raccourci les éléments de liaison ou simplifié les connexions. C'est la création d'un ensemble commun original qui, à l'aide d'un très petit nombre d'éléments, permet de réaliser toutes les fonctions que peuvent exécuter indépendamment chacun des organes cités plus haut.

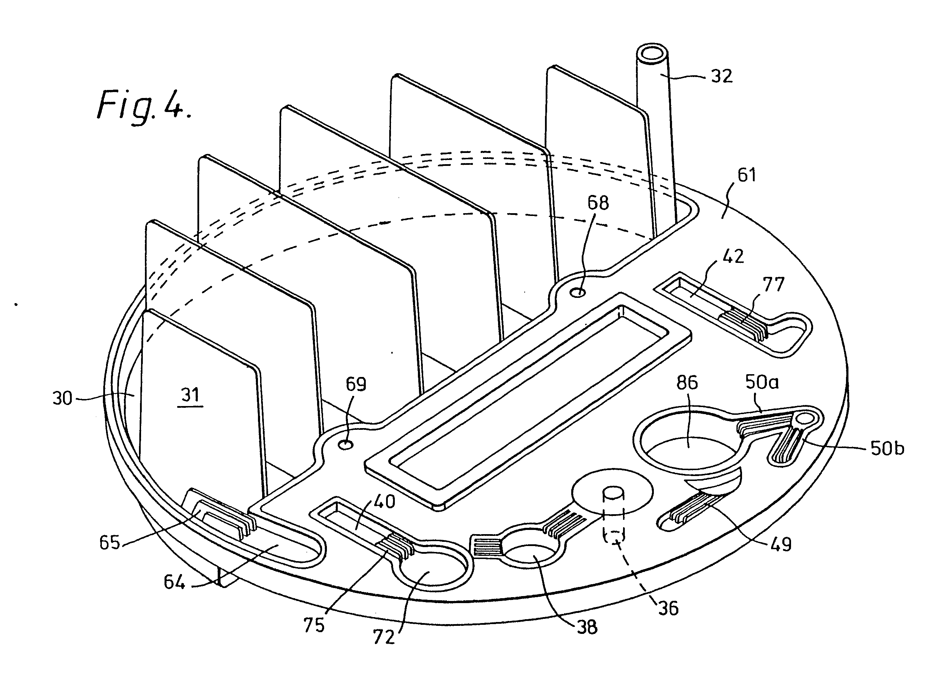

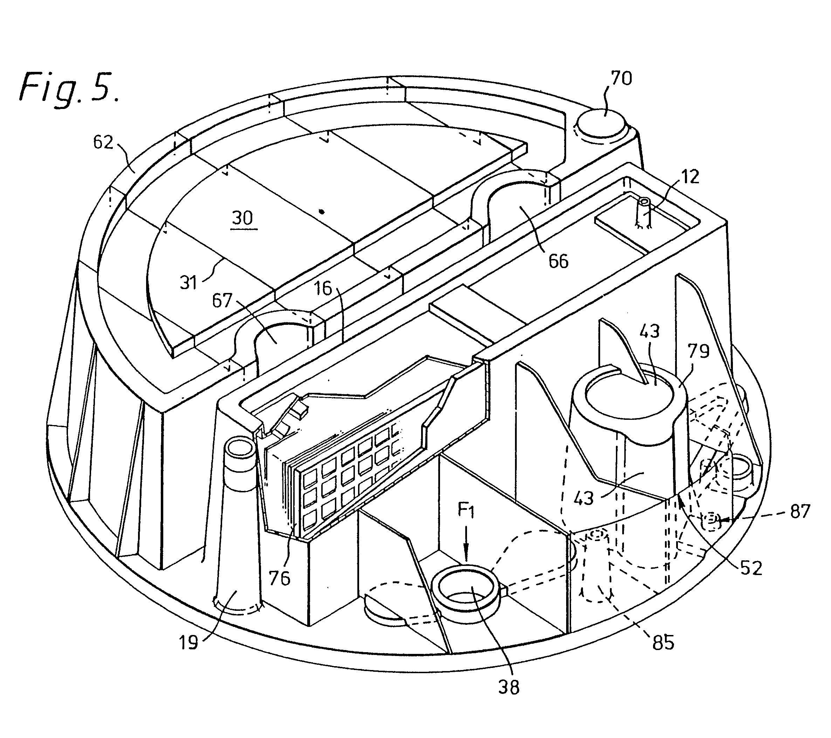

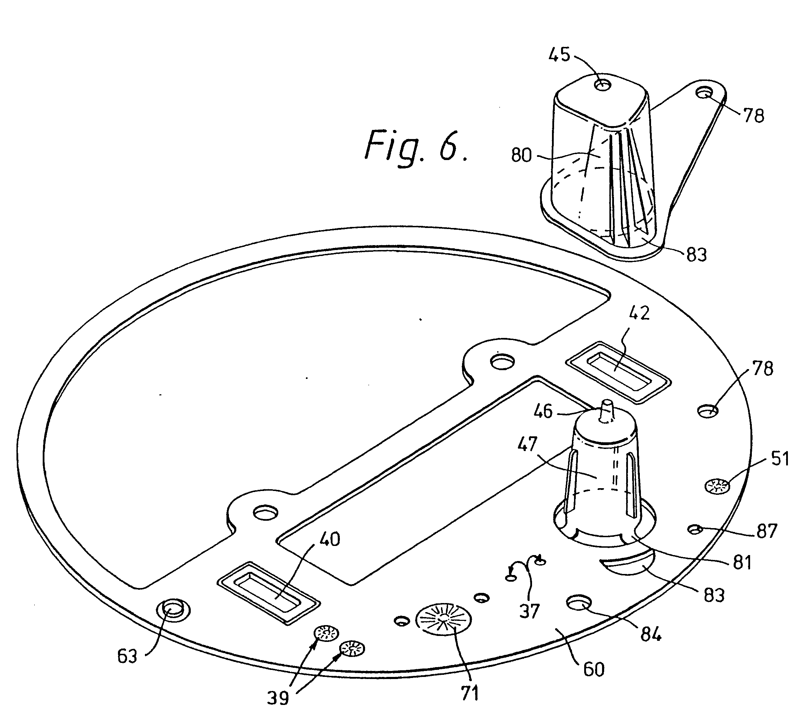

On a trouvé en outre que préférentiellement toutes ces fonctions peuvent être exécutées essentiellement à l'aide d'un élément (60), flexible et souple, sensiblement plan, de faible épaisseur, représenté figure 6, maintenu serré entre deux éléments rigides ou semi-rigides (61) et (62), représentés respectivement figures 4 et 5, ayant des formes déterminées par les fonctions à assurer. Chacun de ces trois éléments est donc multifonctionnel. Ainsi l'élément souple (60) a pour fonction essentielle d'assurer les étanchéités, notamment au contact du liquide de dialyse et en coopération avec les éléments (61) et (62), mais il a localement des fonctions particulières qui seront indiquées plus loin.

En se référant à la figure 4, l'élément inférieur (61), réalisé de préférence en matière plastique injectable rigide telle que le chlorure de polyvinyle ou le polyéthylène, a la forme générale d'un disque ajouré. Si l'on se réfère aux figures 4, 5 et 6 on aperçoit en avant l'embout (19) [figure 5] destiné à être relié au raccord (29) et par là, à l'installation fixe de distribution d'eau. Après remplissage du bac (30) par la solution de concentré de dialyse, cet embout (19) peut recevoir un bouchon que l'on remplace, avant utilisation, par un conduit souple relié au raccord (29). L'eau traverse l'orifice (63) de l'élément (60) [figure 6] qui a localement une fonction de joint d'étanchéité entre les éléments (61) et (62), puis elle pénètre par le canal (64) [figure 4] muni d'ailettes (65) de soutien du joint (60) dans le réservoir (30).

Le fond du réservoir (30) est constitué par environ la moitié du disque (61). Une première série de chicanes (31) est solidaire du fond. La partie correspondante de l'élément supérieur (62) constitue les parois latérales, le couvercle, ainsi qu'une deuxième série de chicanes de ce réservoir. Ces chicanes (31) sont multifonctionnelles puisqu'elles participent en outre à la rigidité de l'ensemble. Les éléments (61) et (62) s'emboîtent exactement de part et d'autre du joint d'étanchéité demi-circulaire constitué par l'élément (60). Les évidements cylindriques (66) et (67) [figure 5] constituent les logements d'appui de la presse de serrage qui fixe par emmanchement à force deux poinçons solidaires de l'élément supérieur (62) dans les orifices cylindriques (68) et (69) correspondant de l'élément inférieur (61). Une fraction du tube plongeur (32) est solidaire de l'élément inférieur (61).

Avantageusement l'élément supérieur (62) [figure 5] comporte au-dessus de l'orifice supérieur du tube (32) un bossage (70) de profil aminci, arrondi, bistable. Enfoncé lors de la fabrication, il obture le tube (32). A l'utilisation, il est soulevé brusquement sous l'effet de la pression de l'eau qui pénètre dans le réservoir (30), libérant ainsi l'accès du liquide de dialyse au container, via le tube (32).

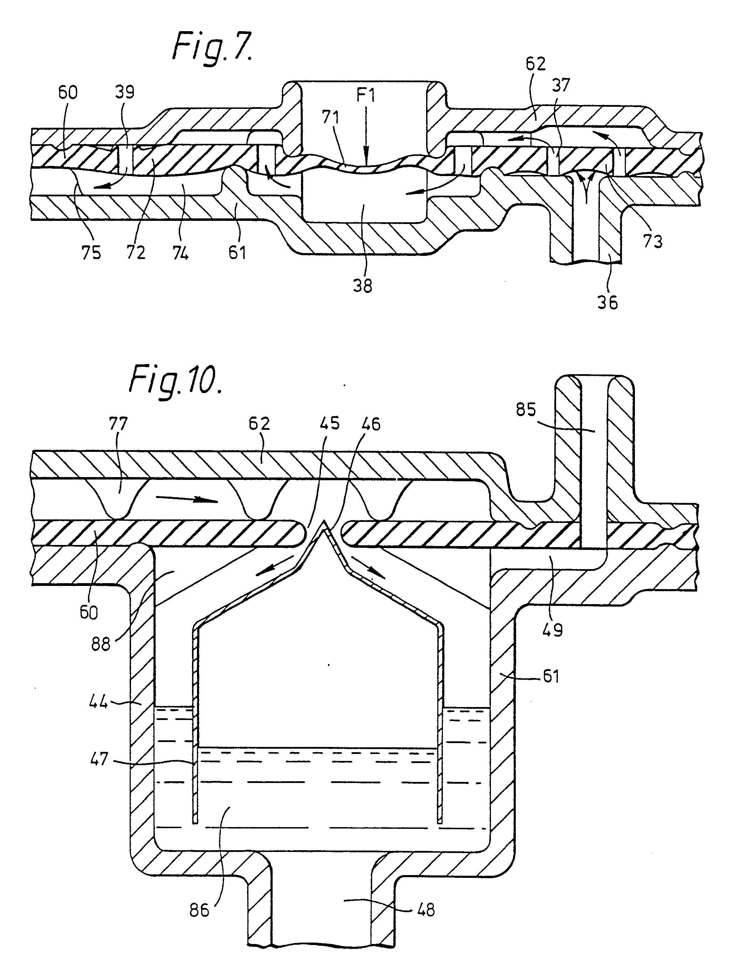

Le liquide de dialyse, stocké dans le compartiment (33) de la poche souple, est repris via le tube (36) [figure 4] par la pompe (38). Cette pompe, du type à membrane, est représentée plus en détails figure 7. La caractéristique essentielle de cette pompe réside dans le fait que l'élément (60) [figure 6] constitue à la fois la membrane, les clapets anti-retour d'admission et de refoulement du liquide de dialyse, ainsi que l'étanchéité périphérique. Cet élément (60) est logé entre les éléments rigides (61) et (62), convenablement conformés pour ménager entre chacun d'eux et la membrane les passages nécessaires au liquide de dialyse. Avantageusement ces passages comportent des nervures telles que (75) [cf. figure 4] pour soutenir l'élément (60). Le déplacement de la partie active (71) de la membrane, convenablement amincie et préformée, est obtenu par une action alternative selon la flèche $F_1$, de tout moyen solidaire de la console (10) tel qu'un motoréducteur de vitesse entraînant une came. Le retour de la partie active de la membrane peut être obtenu, soit par l'action antagoniste d'un ressort mécanique, soit préférentiellement par l'élasticité de la membrane elle-même, soit encore par le fait que la membrane est localement rendue solidaire de la came. Lorsque la partie active (71) de la membrane se déplace selon la flèche $F_1$, le liquide de dialyse est chassé vers l'aval et pousse vers le bas la portion (72) de la membrane constituant le clapet de refoulement, libérant l'orifice de refoulement (39). Lorsque la partie active (71) de la membrane se déplace en sens inverse de $F_1$, la dépression créée notamment à l'amont, élève la portion (73) de la membrane constituant le clapet d'admission, permettant l'introduction dans la pompe (38) par les orifices (37), du liquide de dialyse arrivant par le tube (36).

Le liquide de dialyse parvient ainsi par le canal (74) muni de nervures (75) de soutien de l'élément (71) jusqu'à l'orifice (40) à l'entrée de l'hémodialyseur (16).

Cet hémodialyseur est de type connu en soi, à empilement de plaques intercalaires (76) pleines ou ajourées en treillis et de membranes planes repliées autour de ces intercalaires. Ces intercalaires et ces membranes sont introduits ensemble dans le boîtier de contention constitué par l'élément rigide supérieur (62) et fermé par mise en place de l'élément rigide inférieur (61), l'étanchéité périphérique entre les compartiments parcourus par le sang d'une part et par le liquide de dialyse d'autre part, ainsi que autour des orifices (40) et (42) étant assurée par l'élément flexible (60), les divers éléments (60), (61) et (62) étant localement munis de nervures ou de surépaisseurs convenables. On remarque que les orifices (40) et (42) pour le liquide de dialyse s'ouvrent sur la face de l'hémodialyseur équipée du joint (60) et que les orifices (12a) et (13a) pour le sang s'ouvrent sur la face opposée.

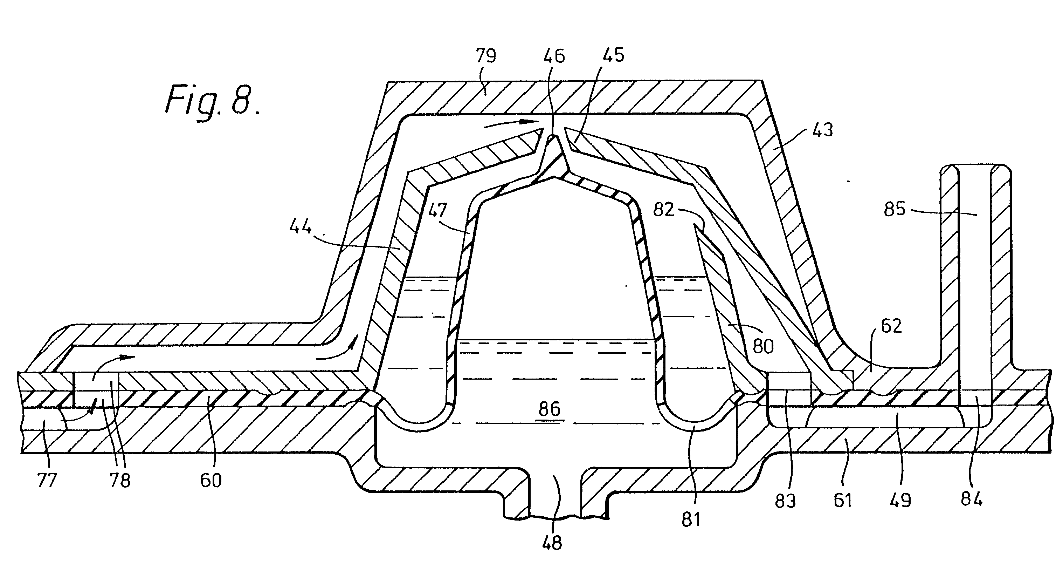

En se référant notamment à la figure 8, le liquide de dialyse usagé qui s'écoule depuis l'orifice (42) le long des nervures (77) traverse l'élément flexible (60) par l'orifice (78) et s'engage sous la forme tronconique (79) de l'élément (62), autour d'une pièce annexe (80) sensiblement coaxiale constituant une partie du bac (44). L'élément tronconique (79) comporte une cannelure (43) en matériau transparent, par exemple en polymétracrylate de méthyle soudé sur l'élément (62), de part et d'autre de laquelle peuvent prendre place les dispositifs émetteur et récepteur de lumière constituant un colorimètre solidaire de la console (10) et qui détecte toute fuite éventuelle de sang dans le liquide de dialyse. Naturellement l'élément (62) peut aussi être entièrement en matériau transparent.

L'élément flexible (60) prend localement la forme d'une cloche reliée par des ailettes (81) et munie au sommet d'un pointeau (46) ; elle constitue le flotteur (47), les ailettes étant assez souples pour laisser au flotteur une liberté de mouvement et un débattement suffisants. Le pointeau (46) vient s'engager dans le siège (45) au sommet de la pièce annexe (80). La pièce (80) est munie d'un déversoir (82) donnant accès par les orifices (83) et (84) et le canal nervuré (49) au conduit (85) de mise à la pression atmosphérique de la chambre réceptrice du flotteur (86). La chambre (86) communique avec le compartiment (35) de stockage du liquide de dialyse usagé par l'orifice (48).

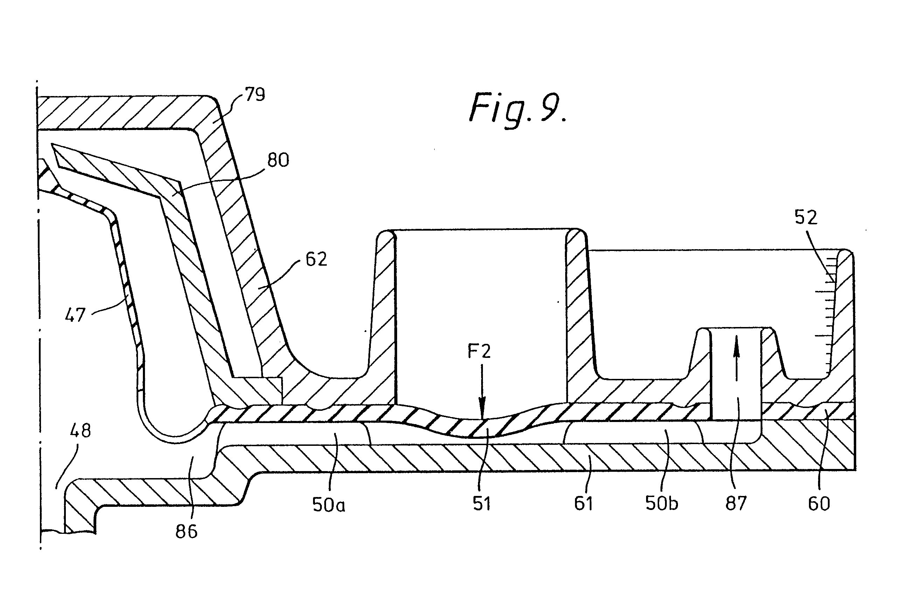

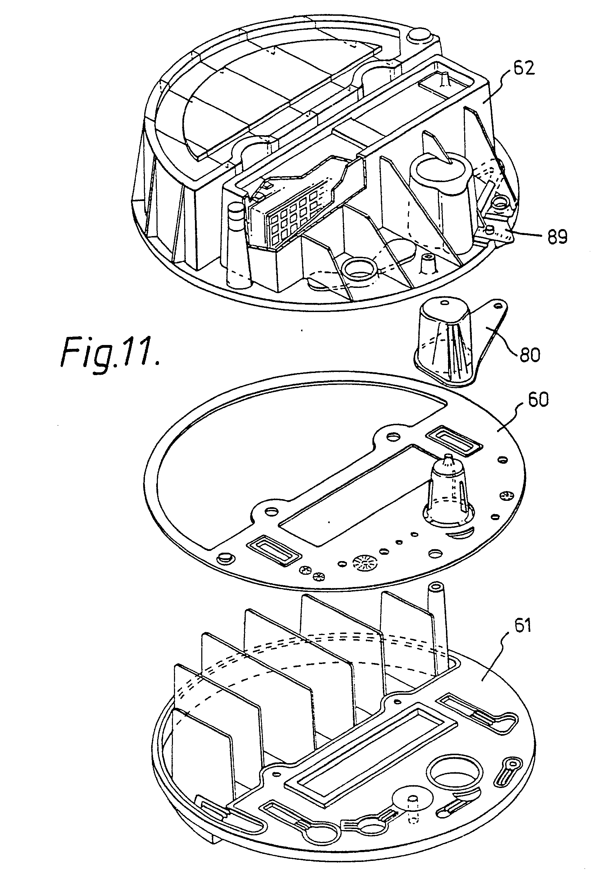

Si l'on se réfère maintenant notamment à la figure 9, on voit que les canaux nervurés (50a) et (50b) conduisent le liquide de dialyse jusqu'à l'orifice (87) d'où il peut s'écouler par débordement dans le récipient gradué (52) limité par une paroi périphérique. La capacité du récipient (52) peut atteindre jusqu'à 3 à 4 litres. Aussi ce récipient peut-il être intégré à l'ensemble commun en étant conformé, soit (comme représenté) par l'élément (62), soit par l'élément (61) qui le dispose en forme d'anneau concentrique autour de l'ensemble commun. Dans ce dernier cas, il peut être alimenté par un déversoir (89) [cf. figure 11].

L'écoulement du liquide de dialyse qui s'effectue par gravité depuis le bac (44) peut être soit arrêté, soit limité et contrôlé par diminution de l'effort permanent exercé sur la portion circulaire déformable (51) du joint (60) selon la flèche F₂, par tout moyen mécanique usuel relié par exemple à la console (10), tel qu'une vis agissant perpendiculairement à la surface (51) du joint. On peut opérer de façon permanente ou de préférence séquentielle en modulant le temps d'ouverture. On peut aussi remplacer ce dispositif par une pompe semblable à la pompe à membrane (38).

Les moyens de stockage du liquide de dialyse sont constitués à l'intérieur du container (20) par les deux compartiments (33) et (35) d'une poche souple, chacun des compartiments étant susceptible d'occuper un volume égal au volume interne du container. Ces compartiments sont formés par exemple à partir d'une gaine tubulaire extrudée en polyéthylène thermosoudée aux deux extrémités et raccordés aux orifices et tubulures correspondants (32), (36) et (48).

De préférence ces moyens de stockage peuvent être intégrés au moins partiellement dans ledit ensemble commun précédemment décrit, par le fait que l'élément inférieur (61) constitue le couvercle (34) du container (20). La gaine tubulaire aplatie couvre alors la face inférieure de l'élément (61). Cette intégration peut être encore accrue par le fait que l'on peut avantageusement introduire la gaine tubulaire aplatie, avec le joint (60), entre les éléments (61) et (62) ou autour de l'élément (62), les orifices appropriés pouvant être exécutés dans le joint (60) et dans l'une au moins des parois de la gaine tubulaire aplatie.

Il ressort de la description ci-dessus que, à l'aide de deux ou trois accessoires logés notamment dans la console (10) et actionnant F₁ et F₂, trois ou quatre éléments seulement constituent un ensemble commun qui permet d'effectuer toutes les fonctions de préparation et de circulation du liquide de dialyse au cours d'une séance d'hémodialyse, ainsi que de contrôle volumétrique de l'ultrafiltrat. En intégrant une poche souple à cet ensemble commun on permet en outre à celui-ci de stocker le liquide de dialyse frais et/ou usagé.

L'ensemble commun ainsi décrit peut être livré avec sa réserve de solution de concentré, sous emballage stérile, prêt à l'emploi. L'utilisateur n'a qu'à disposer cet ensemble sur le container (20), le relier au circuit sang et mettre en place la console (10).

Il est tout à fait remarquable de pouvoir exercer des fonctions aussi nombreuses et variées à l'aide d'un nombre de pièces aussi considérablement réduit. Un rein artificiel avec circuit de dialyse intégré présente notamment les avantages suivants :

a) mise en œuvre de moins de pièces et de moins de matière, donc économie d'énergie, de fabrication, de montage, de transport, de stockage et d'utilisation. En particulier la suppression de la plupart des connexions, la grande compacité de l'ensemble intégré et le cas échéant, le calorifugeage de la console (10) et du container (20) éliminent pour l'essentiel les pertes thermiques en cours de séance, ce qui permet de simplifier considérablement les dispositifs de chauffage et de régulation de la température du sang, tout en conservant une très grande sécurité.

b) l'emploi à usage unique du circuit de dialyse devient ainsi nettement plus accessible, d'où :

des fabrications de grande série se prêtant bien à être automatisées, et de remarquables facilités d'assemblage ce qui accroît encore l'économie,

une faible durée de vie autorisant l'emploi de matériaux bons marchés et un sensible allègement des contraintes de fabrication,

la suppression de toute stérilisation et des accessoires qui lui sont nécessaires.

c) mise en œuvre rapide, simple et sûre : plus de stérilisation, pas de raccordements préalables et moins de contrôles à effectuer.

Tous ces avantages sont particulièrement appréciables pour la dialyse à domicile.

Comme indiqué plus haut, la présente invention peut faire l'objet d'un grand nombre de variantes de réalisation à la portée du technicien. Seulement à titre d'exemples nous citerons quelques unes de ces variantes.

Ainsi le bossage bistable (70) peut être supprimé : en effet le tube (32) ne débouche que dans un compartiment (33) préalablement vide d'air qui ne peut donc recevoir, avant utilisation, qu'un volume de solution concentré négligeable. .

L'hémodialyseur (16) peut être du type à fibres creuses.

Le dispositif à flotteur peut être aussi réalisé comme représenté figure 10. La pièce annexe est ici le flotteur (47) muni du pointeau (46). Le bac (44) est constitué par l'élément inférieur (61) convenablement conformé. Il est recouvert par l'élément flexible (60), muni d'une ouverture (45) constituant le siège de l'obturateur et il est soutenu par des nervures radiales (88). La chambre (86) est reliée à l'atmosphère par les conduits (49) et (85).

Le dispositif de prélèvement de liquide pour le contrôle de l'ultrafiltration peut être relié directement au compartiment (35) récepteur du liquide de dialyse usagé.

La poche souple peut n'être constituée que par un seul compartiment, soit pour le liquide de dialyse frais, soit pour le liquide de dialyse usagé. Le container doit alors être muni de joints d'étanchéité. On peut aussi ne pas utiliser de poche souple, laisser le liquide de dialyse directement dans le container qui devient à usage unique, un certain mélange des liquides frais et usagé n'étant alors pas exclu. Le cas échéant on peut alors intégrer une ou plusieurs cartouches d'adsorbants dans l'ensemble commun précédemment décrit.

Il a bien été précisé que l'on ne sort pas du cadre de la présente invention si l'on ne procède qu'à une intégration partielle des différents organes du circuit de liquide de dialyse. Ainsi, uniquement à titre d'exemples, on peut, dans le cadre de la présente invention, n'intégrer que la pompe (38) à l'hémodialyseur (16), le bac de solution concentré (30) restant autonome. De même le container (20) est intégré à l'ensemble commun même si seul le couvercle (34) constitue l'un des éléments de l'ensemble commun.

## Revendications

1. Rein artificiel comprenant :

a) un hémodialyseur (16) comportant un boîtier séparé en deux compartiments, le premier muni de moyens pour la circulation du sang et le second pour la circulation du liquide de dialyse, par une membrane (41) permettant le traitement du sang par dialyse et par ultrafiltration,

b) des moyens extérieurs audit hémodialyseur pour faire circuler le sang dans ledit premier compartiment,

c) des moyens extérieurs audit hémodialyseur, pour préparer le liquide de dialyse et le faire circuler,

d) des moyens pour stocker ledit liquide de dialyse, frais et/ou usagé,

e) des moyens pour prélever et mesurer des quantités de liquide égales aux quantités d'ultrafiltrat désirées et,

f) des organes de commande et de contrôle desdits moyens selon b) et c),

lesdits moyens selon b, c, d et/ou e étant essentiellement constitués de trois éléments non permanents, à savoir un élément flexible (60) ayant la forme générale d'une feuille plane, localement ajourée, comprise à l'intérieur et maintenue serrée entre deux éléments rigides ou semi-rigides (61, 62), ces trois éléments coopérant ensemble pour, d'une part délimiter les circuits de liquide entre l'élément flexible et les éléments rigides, à l'intérieur et au contact desdits éléments rigides ou semi-rigides et à l'extérieur dudit élément flexible (60), lesdits circuits étant susceptibles de communiquer à travers ledit élément flexible, et pour, d'autre part, assurer les étanchéités.

2. Rein artificiel selon la revendication 1, caractérisé en ce que l'un au moins desdits deux éléments rigides ou semi-rigides (61, 62) constitue au moins en partie les moyens de contention dudit hémodialyseur (16).

3. Rein artificiel selon l'une quelconque des revendications 1 ou 2, caractérisé en ce que ledit élément flexible (60) constitue l'une des parties actives d'un dispositif obturateur à flotteur permettant de réguler la pression du liquide de dialyse dans l'hémodialyseur.

4. Rein artificiel selon l'une des revendications 1, 2 caractérisé en ce que ledit élément flexible (60) constitue une partie active d'un dispositif d'évacuation du liquide de dialyse permettant un contrôle volumétrique de l'ultrafiltration.

## Claims

1. Artificial kidney comprising :

a) a haemodialyser (16) comprising a casing divided into two compartments, the first equipped with means for circulating the blood and the second for circulating the dialysis liquid, by a membrane (41) permitting the blood to be treated by dialysis and by ultrafiltration,

b) means external to the said haemodialyser for causing the blood to circulate in the said first compartment,

c) means external to the said haemodialyser for preparing the dialysis liquid and for causing it to circulate,

d) means for storing the said dialysis liquid, fresh and/or used,

e) means for withdrawing and measuring quantities of liquid equal to the required quantities of ultrafiltrate, and

f) instruments for controlling and for monitoring the said means according to b) and c), the said means according to b, c, d and/or e consisting essentially of three temporary members, namely a flexible member (60) having the overall shape of a plane sheet, locally perforated, included inside and held clamped between two rigid or semirigid members (61, 62), these three members interacting together in order, on the one hand, to define the liquid circuits between the flexible member and the rigid members, inside and in contact with the said rigid or semirigid members and outside the said flexible member (60), the said circuits being capable of communicating through the said flexible member, and in order, on the other hand, to provide the sealing.

2. Artificial kidney according to Claim 1, characterized in that at least one of the said two rigid or semi-rigid members (61, 62) forms at least partly the means for retaining the said haemodialyser (16).

3. Artificial kidney according to either of Claims 1 and 2, characterized in that the said flexible member (60) forms one of the working parts of a float valve device permitting the pressure of the dialysis liquid in the haemodialyser to be regulated.

4. Artificial kidney according to one of Claims 1 and 2, characterized in that the said flexible member (60) forms a working part of a device for removing the dialysis liquid and permitting a volumetric monitoring of the ultrafiltration.

**Patentansprüche**

1. Künstliche Niere, enthaltend

a) einen Hämodialysator (16) mit einem in zwei Abteile unterteilten Gehäuse, von denen das erste Mittel für die Zirkulation des Blutes und das zweite Mittel für die Zirkulation der Dialyseflüssigkeit aufweisen, wobei das Gehäuse durch eine Membran (41) unterteilt ist, die die Behandlung des Blutes durch Dialyse und durch Ultrafiltration gestattet,

b) außerhalb des Hämodialysators angeordnete Mittel, um das Blut in das erste Abteil zirkulieren zu lassen,

c) außerhalb des Hämodialysators angeordnete Mittel, um die Dialyseflüssigkeit aufzubereiten und zirkulieren zu lassen,

d) Mittel zum Speichern der Dialyseflüssigkeit im frischen und/oder benutzten Zustand,

e) Mittel zum Entnehmen und Messen von Dialyseflüssigkeitsmengen, die den gewünschten Ultrafiltratmengen gleich sind, und

f) Organe zum Steuern und Kontrollieren der Mittel b) und c),

wobei die Mittel b), c), d) und/oder e) im wesentlichen aus drei nicht beständigen Elementen bestehen, nämlich einem biegsamen Element (60) mit der allgemeinen Form eines ebenen, örtlich durchbrochenen, innerhalb enthaltenen und zwischen zwei starren oder halbstarren Elementen (61, 62) geklemmt gehaltenen Blattes, wobei diese drei Elemente zusammenarbeiten, um einerseits die Flüssigkeitskreise zu begrenzen zwischen dem biegsamen Element und den starren Elementen innerhalb und in Berührung mit den starren oder halbstarren Elementen und außerhalb des biegsamen Elements (60), wobei die Kreise durch das biegsame Element hindurch miteinander in Verbindung stehen können, und um andererseits die Abdichtungen zu gewährleisten.

2. Künstliche Niere nach Anspruch 1, dadurch gekennzeichnet, daß wenigstens das eine der beiden starren oder halbstarren Elemente (61, 62) wenigstens zum Teil die Aufnahmemittel des Hämodialysators (16) bildet.

3. Künstliche Niere nach einem der Ansprüche 1 oder 2, dadurch gekennzeichnet, daß das biegsame Element (60) einen der aktiven Teile einer Verschlußvorrichtung mit Schwimmer bildet, die ein Einstellen des Drucks der Dialyseflüssigkeit im Hämodialysator gestattet.

4. Künstliche Niere nach einem der Ansprüche 1, 2, dadurch gekennzeichnet, daß das biegsame Element (60) einen aktiven Teil einer Vorrichtung zum Entleeren der Dialyseflüssigkeit bildet, die eine volumetrische Kontrolle der Ultrafiltration gestattet.

Fig.1.

Fig. 2 .

Fig. 3.

0 052 571

Fig.4.

*Fig.5.*

0 052 571

Fig. 6.

## Fig.7.

## Fig.10.

Fig. 8.

0 052 571

*Fig.9.*

0 052 571

*Fig.11.*